# EUROPEAN PATENT SPECIFICATION

(11) **EP 1 711 631 B1**
(45) Date of publication and mention of the grant of the patent: **30.09.2009**
(21) Application number: 05701850.9
(22) Date of filing: 12.01.2005
(51) Int. Cl.: C12Q 1/68

(54) **NUCLEIC ACID CHARACTERISATION**
NUKLEINSÄURECHARAKTERISIERUNG
CARACTERISATION D'ACIDES NUCLEIQUES

(30) Priority: 12.01.2004 GB 0400584
(43) Date of publication of application: 18.10.2006
(73) Proprietor: Illumina Cambridge Limited, Little Chesterford Saffron Walden Essex CB10 1XL (GB)
(72) Inventor: GORMLEY, Niall Anthony Solexa Limited, Saffron Walden Essex CB10 1XL (GB); McCOOKE, Nicholas John Solexa Limited, Saffron Walden Essex CB10 1XL (GB)
(74) Representative: White, Nina Louise
(86) International application number: PCT/GB2005/000080
(87) International publication number: WO 2005/068656

(56) References cited:
- WO-A-98/44151
- WO-A-02/061143
- US-A- 5 770 365
- US-B1- 6 403 319

### SEQUENCE LISTING

<110> Solexa Limited
<120> Nucleic Acid Characterisation
<130> P64923WO00
<160> 8
<170> PatentIn version 3.1
<210> 1
   <211> 21
   <212> DNA
   <213> Artificial sequence
<220>
   <223> 5' strand of hairpin primer A
   <400> 1
   atctatccga ctcgcaggtt t 21
<210> 2
   <211> 22
   <212> DNA
   <213> Artificial sequence
<220>
   <223> 3' strand of hairpin primer A
<400> 2
   ttacctgcga gtcggataag at 22
<210> 3
   <211> 21
   <212> DNA
   <213> Artificial sequence
<220>
   <223> 5' strand of hairpin primer B
<400> 3
   tcatatccga ctcgcaggtt t 21
<210> 4
   <211> 21
   <212> DNA
   <213> Artificial sequence
<220>
   <223> 3' strand of hairpin primer B
<400> 4
   ttacctgcga gtcggatatg a 21
<210> 5
   <211> 17
   <212> DNA
   <213> Artificial sequence
<220>
   <223> Strand of Template 1
<400> 5
   tcttggagtg gtgaatc 17
<210> 6
   <211> 19
   <212> DNA
   <213> Artificial sequence
<220>
   <223> Strand of template 1
<400> 6
   cggattcacc actccaaga 19
<210> 7
   <211> 17
   <212> DNA
   <213> Artificial sequence
<220>
   <223> Strand of Template 2
<400> 7
   cgcttcgtta atacaga 17
<210> 8
   <211> 19
   <212> DNA
   <213> Artificial sequence
<220>
   <223> Strand of Template 2
<400> 8
   catctgtatt aacgaagcg 19

## Description

### Field of the invention

The present invention is concerned with nucleic acid characterisation and in particular the use of nucleic acid arrays in such characterisation and analysis.

### Background to the invention

The study of complex genomes, in particular, the search for the genetic basis of disease in humans requires genotyping on a massive scale. Screens for numerous genetic markers performed for populations large enough to yield statistically significant data are needed before associations can be made between a given genotype and a particular disease. However, large-scale genotyping is demanding in terms of cost, time and labour, especially if the methodology employed involves serial analysis of individual DNA samples, i.e., separate reactions for individual samples. One shortcut is to pool DNA from many individuals and to determine parameters such as the frequencies of a genotype, *e.g.*, an allele, among the individuals and then to correlate the frequency of an allele in an affected population with the occurrence of a disease. Hence, an association study involving 1000 patients would in theory only necessitate a 'one-pot' reaction. Pooling therefore represents an effective technique for analysing large quantities of samples in a facile manner.

One disadvantage of pooling samples prior to analysis is that information pertaining to individual DNA samples is lost: only global information such as allele frequencies is gathered. There is no easy method of discerning which individuals gave rise to a particular genotype. An ability to genotype large populations in a small number of reactions while retaining the information relating to individual samples would yield the information of a full 'non-pooled' population screen at the cost of a few pooled reactions.

DNA from more than one source can be sequenced on an array if each DNA sample is first tagged to enable its identification after it has been sequenced. Many different DNA-tag methodologies already exist ranging from: ESTs (short sequences derived from cDNAs that map the position of expressed genes in a genome, Adams et al., (1991) Science 252, 1651) to simple fluorescent dyes for labelling (Haugland, R., Handbook of Fluorescent Probes and Research Products, 9th edition, Molecular Probes). Other methods include the use of: branched nucleic acid dendrimers (US Pat. 6504019), quantum dots (Bruchez, M.P. et al., (1998) Science 281, 2013) and combinatorial nucleic acid words (US Pat. 5604097). In the last reference, DNA tags are added to the ends of genomic DNA fragments by cloning. The tags consist of eight four-base 'words', where each word uses only three bases (A,T, and C) in various combinations resulting in a total of 16,777,216 different tags that all have the same base-pair composition and identical melting points. The tagged DNA fragments are substrates for analysis of gene expression on microbead arrays (Brenner et al., (2000) Nat. Biotech, 18, 630). These combinatorial DNA tags are more applicable to tagging large numbers of DNA samples in comparison to physical tags, such as fluorescent molecules, because of the size of the tag repertoire. Furthermore, nucleic acid based tags are more amenable to manipulation by standard molecular biology protocols, such as PCR or endonuclease cleavage.

### Summary of the invention

The present invention employs tags comprising defined sequences of nucleic acid bases to tag polynucleotide molecules derived or isolated from a plurality of different sources, such as for example molecules isolated different individuals.

Therefore, in-accordance with the present invention there is provided a method of sequencing and distinguishing between nucleic acid sequences on an array, which sequences originate from different sources, which method comprises the steps of,
a) ligating target nucleic acid sequences comprising DNA polynucleotides from a first source to double-stranded nucleic acid anchoring molecules thereby producing tagged target nucleic acid sequences, wherein said double-stranded anchoring molecules comprise a nucleic acid sequence tag characteristic of the target nucleic acid sequence source and a functionality capable of effecting immobilization to said array, and wherein the 3' end of each strand of said DNA polynucleotide is ligated to the 5' end of one strand of said double-stranded anchoring molecule, which 5' end is not used for anchoring;
b)repeating step a) for target nucleic acid sequences from second and subsequent sources using second and subsequent nucleic acid anchoring molecules having characteristic nucleic acid sequence tags for each of said sources;
c) immobilising tagged target nucleic acid sequences from different sources to said array via the functionality capable of effecting immobilisation, thereby producing immobilised molecules, each immobilised molecule comprising a target nucleic acid sequence and a nucleic acid sequence tag characteristic of the target nucleic acid sequence source, wherein said nucleic acid sequences on said array are disposed at a density such that they are capable of individual resolution using optical microscopy; and
d) sequencing said immobilised individually resolvable molecules by addition of nucleotides to a 3' hydroxyl group on the immobilised primer, whereupon said sequencing identifies a sequence of each of the target nucleic acid molecules and the sequence of the tag to identify the corresponding source of the target nucleic acid molecule.

The present invention represents an advance in array technology whereby pooled target nucleic acids from a plurality of sources can be sequenced on a single array and the origin of the target nucleic acids identified. As described herein, the presence of a characteristic nucleic acid sequence tag on an immobilised molecule comprising a target nucleic acid sequence permits the source of the target nucleic acid to be identified concurrently with the sequencing of said nucleic acid. The term " distinguishing between" nucleic acid sequences on an array therefore refers to distinguishing between nucleic acid sequences on the array which originate from different sources. This is a dramatic improvement over pre-existing array technologies which generally require an initial sequencing step for sequencing the pooled nucleic acid, followed by a subsequent step wherein the source of the nucleic acid is determined.

As will be apparent to the skilled reader, references herein to a particular nucleic acid sequence may, depending on the context, also refer to nucleic acid molecules which embody the nucleic acid sequence.

The present invention will now be further described. In the following passages different aspects of the invention are defined in more detail. Each aspect so defined may be combined with any other aspect or aspects unless clearly indicated to the contrary. In particular any feature indicated as being preferred or advantageous may be combined with any other feature or features indicated as being preferred or advantageous.

The terms "target nucleic acid sequence", "target nucleic acid molecule", "target nucleic acid" and "target nucleic acid fragment" may be used interchangeably to refer to nucleic acid molecules that it is desired to sequence on an array according to the invention. The target nucleic acid may be essentially nucleic acid of known or unknown sequence. It may be, for example, a fragment of genomic DNA or cDNA. Sequencing may result in determination of the sequence of a whole or a part of the target molecule.

The method of the invention utilises "nucleic acid sequence tags" as markers characteristic of the source of particular target nucleic acid molecules on the array. A nucleic acid sequence tag characteristic of source is attached to each of the target nucleic acid molecules immobilised on the array. The tag is not itself formed by part of the target nucleic acid molecule or derived from the target molecule, meaning that the tag is not a sequence contiguous with the target nucleic acid sequence when the latter is in its natural context. Generally the tag will be a synthetic sequence of nucleotides which is added to the target nucleic acid prior to or during immobilisation on the array.

Preferably, the nucleic acid sequence tag may be up to 100 nucleotides (base pairs if referring to double stranded molecules) in length, more preferably from 1 to 10 nucleotides in length, and most preferably 4, 5 or 6 nucleotides in length. Different tags may comprise different combinations of nucleotide characteristic of a given source of target nucleic acids.

In one embodiment of this aspect of the invention, the capture moiety itself comprises a nucleic acid sequence which can be immobilised on the surface of the array and this capture moiety preferably comprises the characteristic nucleic acid sequence tag. The capture moiety comprises a double stranded nucleic acid molecule. In one embodiment this may comprise, for example, a hairpin oligonucleotide, to which the nucleic acid sequence is covalently attached.

A number of different embodiments of the capture moiety may therefore be utilised in accordance with the method of the invention. In a first aspect, the double stranded nucleic acid molecule may comprise first and second ends one end of which will be for attachment to the target nucleic acid molecule, the other being for anchoring to the array. Hence, the capture moiety may be referred to as a "double stranded nucleic acid anchoring molecule". In this context "anchoring" is taken to mean immobilisation of a molecule incorporating the anchoring molecule on the array.

The capture moiety may comprise a 5' or 3' overhanging sequence at one of its ends. In a first embodiment of this aspect the overhang may be provided on the 5' end of one of said strands relative to the 3' end of the complementary strand thereof and which 5' overhanging sequence may comprise the nucleic acid sequence tag, which tag is characteristic of a particular nucleic acid source. Where the double stranded molecule includes said 5' overhang, target nucleic acid, which is preferably DNA, from a particular source may be covalently attached to the 5' overhanging end of the double stranded capture moiety using an appropriate ligation reagent such as, for example a ligase enzyme, preferably a DNA ligase. In this embodiment a single stranded DNA molecule may be ligated to said 5' end. The 3' end of the double stranded capture moiety that is complementary to the 5' end of the strand having the target nucleic acid molecule ligated thereto may thus act as a primer sequence in a DNA resequencing protocol to identify the sequence of the 5' overhang which functions as the template and includes both the nucleic acid sequence tag associated with the 5' overhanging portion, in addition to the DNA ligated thereto.

The term "ligation reagent" encompasses any reagent capable of effecting or catalysing ligation between two nucleic acid strands. Suitable ligation reagents include ligase enzymes, such as DNA ligase. Different ligase enzymes have the ability to ligate different types of single-stranded or double-stranded DNA and/or RNA, as would be apparent to the skilled reader.

As would be known to the skilled practitioner, a ligase enzyme requires the presence of a phosphate molecule at the 5' end of the molecule to which it is to be ligated, in order to ligate the DNA sequence thereto, such a phosphate moiety may be provided on the 5' end of the double stranded nucleic acid capture moiety to which the target nucleic acid sequence is to be attached. Thus, the sequence of bases including the characteristic tag sequence on the 5' template overhanging strand may be determined by employing a polymerase enzyme to synthesise a complementary strand to the template DNA one base at a time. Each added base preferably comprises a characteristic fluorophore attached that permits its identification by an appropriate detection means and the next base can be similarly identified once the fluorophore is removed. While any suitable DNA sequencing method may be utilised, as would be known to one of skill in the art, a preferred method involves DNA resequencing methodology, as described in US 5,302,509.

Alternatively, the capture moiety may not contain any overhang, the nucleic acid tag being provided in the sequence of the double stranded capture moiety. Accordingly, in one embodiment double stranded DNA (e.g. genomic DNA) may be covalently attached or otherwise ligated to-one end of the double stranded nucleic acid capture moiety. In one such an embodiment, only the 5' end of the strand to which the target nucleic acid is to be attached may include the phosphate moiety, and accordingly the ligase will only ligate a single strand of the genomic DNA to be tested to this 5' end. Thus, the genomic DNA may preferably be pretreated (e.g. with phosphatase) to remove any phosphate moiety from its 5' and 3' ends. In such an embodiment, the non-contiguous strand of the genomic DNA may be removed according to procedures known in the art, thus leaving a single strand attached to the 5' end of one of the strands of the capture moiety. The 3' end of the complementary strand of the capture moiety may then function as a primer sequence to sequence the target DNA attached to the 5' end of the capture moiety in the manner described above.

Therefore, in one embodiment, the nucleic acid tag is provided as a single strand on an overhanging portion on the 5' end of the strand to which the target nucleic acid is to be attached, together with the target nucleic acid sequence. The corresponding 3' end of the complementary strand may be used as a primer for extension of the 3' strand in the 5' to 3' direction using the complementary strand at the 5' end as a template resulting in sequencing and therefore identification of the target nucleic acid sequence and the tag.

In further embodiments the double stranded capture moiety by be blunt-ended, i.e with no 5' overhang, at the end to which the target nucleic acid molecule is to be ligated. The tag sequence may be present at the 5' end of the strand to which the target molecule is to be ligated. A complement to the tag sequence will be present on the other strand (referred to herein as the 3' strand) of the double stranded molecule. Following ligation of the target nucleic acid molecule to the 5' end of the double stranded capture moiety, a portion of the 3' strand may be cleaved and removed in order to create a 5' overhang, thereby providing a template ready for sequencing. This cleavage step may remove the region of the 3' strand complementary to the tag, thereby exposing the tag for sequencing. In such embodiments cleavage of the 3' strand generates a 3' hydroxyl group which provides an initiation point for sequencing.

In order to direct cleavage of the 3' strand, the double stranded capture moiety may comprise an endonuclease recognition sequence and a cleavage site. Preferably, the nucleic acid sequence tag and the endonuclease recognition sequence are oriented with respect to each other such that the endonuclease is capable of cleaving or nicking at the cleavage site on the 3' strand at a nucleotide position that is upstream (i.e. 5') of, and preferably immediately adjacent to (or up to but not including) a nucleotide of complement of the nucleic acid sequence tag on the 3' strand. By "immediately adjacent to" is meant that the enzyme cleaves at a phosphodiester bond formed between the 5' phosphate of the first nucleotide forming the tag sequence and the 3' hydroxyl group of the preceding nucleotide. However, the recognition and cleavage sites may be designed in said capture moiety such that the endonuclease cleaves at any position on the 3' strand complementary to the strand to which the target nucleic acid is attached so as to remove the sequence complementary to the nucleic sequence tag (optionally together with further upstream nucleotides from the 3' strand). Therefore, the tag is exposed on the 5' strand and the remainder of the 3' strand may as a primer for the sequencing-by-synthesis of the complementary strand in the 5' to 3' direction. Preferably cleavage will occur at a position not more than 5 and more preferably not more than 2 nucleotides upstream of the nucleic acid tag, and most preferably immediately adjacent to the tag. If cleavage occurs immediately upstream of the complement of the tag sequence on the 3' strand then the first bases sequenced in such a sequencing reaction will be the tag sequence.

Accordingly, once the endonuclease has nicked or cleaved the complementary strand to remove the complement of the tag sequence, the remaining 3' end of the nicked strand on the double stranded capture moiety may again function as a primer in a polymerase based resequencing reaction to identify both the nucleic acid sequence tag and the ligated target nucleic acid sequence in a single sequencing protocol.

The method involves determining sequence of a portion of the target nucleic acid and the sequence of the tag characteristic of the source of the target nucleic acid in a single sequencing reaction step. Such embodiments may require a cleavage step prior to sequence in which the 3' strand of the capture moiety is nicked or cleaved by an endonuclease at a cleavage site positioned so the cleaved portion of 3' strand that is removed at least includes the complement of the nucleic acid sequence tag. Such cleavage thus exposes the sequence tag for sequencing and generates a 3' end for initiation of a sequencing-by-synthesis reaction. The sequencing reaction will first determine the sequence of the characteristic tag, followed by a portion of the target nucleic acid molecule.

Alternatively a two step sequencing procedure may be utilised whereby the target nucleic acid attached to the double stranded capture moiety at the 5' end of one of the strands is first sequenced by virtue of the end of the 3' strand acting as a primer. Sequencing can proceed simply by addition of further nucleotides to the 3' end of this strand. Such sequencing will result in determination of the sequence of a portion of the target nucleic acid. The complementary (3') strand may then subsequently be cleaved to reveal or expose a single stranded nucleic acid sequence tag and which may then be subsequently sequenced in a second sequencing reaction to identify the source of the nucleic acid.

As would be known to one of skill in the art, a nicking endonuclease is one of a class of enzymes that bind reversibly to a specific recognition sequenc in a double stranded nucleic acid and cleaves a phosphodiester bond in only one strand at a cleavage site located short distance from the recognition site. The result is a "nick" in one strand rather than a cleavage of both strands. In general the nicks occur at the 3'hydroxyl, 5' phosphate, therefore cleavage generates a free 3' hydroxyl group. When a nick is produced in a section of double stranded nucleic acid, the portion of the nicked strand distal to (downstream of) the cleavage site is no longer continuous with the main body of the double stranded nucleic acid. It becomes, in essence a -single stranded molecule hybridised to the rest of the nucleic acid and can therefore be removed by procedures known in the art.

The restriction site for a given endonuclease comprises both a recognition sequence and a cleavage site. The recognition sequence is the precise sequence of nucleotides recognised by a particular endonuclease. The recognition sequence for the endonuclease N.BstNBI is GAGTCNNNN, where N can be any nucleotide. The cleavage site for this endonuclease is four nucleotides 3' from the end of this recognition sequence. Therefore, the restriction site can be oriented in the capture moiety to ensure that the nicking or cleavage of the 3' strand embraces all of the complementary tag sequences on the 3' strand. There is no requirement that the restriction site be situated so that the endonuclease cuts or nicks exactly at the nucleotide on the 3' strand immediately before the complement of the tag sequence. The cleavage site can be positioned at any point to ensure that the endonuclease cuts or nicks upstream (5') of a sequence that comprises the tag sequences in the 3' strand. Thus any appropriate endonuclease can be used.

For example, there exist nicking endonucleases that nick or cleave at a position 3' of the recognition sequence, that is, the recognition sequence and the cleavage site are separated by several nucleotides. Such nicking endonucleases include N.AInI, N.BspD6I, N.Bst9I, N.BstNBI, N.BstSEI, where four random nucleotides separate the recognition sequence and the cleavage site, and N.MlyI, where time random nucleotides separate the recognition and the cleavage site.

There is no requirement that the recognition sequence be separated from the cleavage site. There exist nicking endonucleases that cut (cleave) within the recognition sequence (eg N.BbvCIB, N.Bpn1OIA, N.Bpn1OIB, N.CviPII, N.CviQXI), similar to the action of an ordinary restriction enzyme, i.e. an enzyme that cleaves through both strands of a double stranded nucleic acid.

Preferably, the double stranded nucleic acid capture moiety comprises a hairpin oligonucleotide. Hairpins including a 5' overhanging sequence portion may be generated by designing said hairpin to have regions of internal self complementarity to encompass the 3' end but with additional nucleotides at the 5' end which do not have complements on the 3' strand. In another embodiment, the hairpin may be "blunt-ended" such that the region of complementarity enables the formation of the intramolecular duplex including each of the 5' and 3' ends of the oligonucleotide, each nucleotide thus having a complementary nucleotide in the other arm of the intermolecular duplex.

The capture moiety according to the invention may be immobilised on an array prior to or subsequent to attachment of the target nucleic acid.

Target nucleic acids from different sources may be immobilised on the array together with different sequence tags, each characteristic of a particular source of nucleic acid. Therefore, nucleic acid from a first source may be added to the array and immobilised thereto using a hairpin oligonucleotide, in the same manner as previously described, having a characteristic nucleic acid sequence tag. Nucleic acids from other sources may also be immobilised using hairpin oligonucleotides incorporating different characteristic nucleic acid sequence tags.

The array comprises an array of single molecules that are capable of being resolved by optical microscopy. An array of single molecules may be prepared in accordance with the methods as disclosed in WO 00 /06770. Single molecule arrays are arrays of molecules, such as polynucleotide molecules immobilised on a surface at a density which allows each of the target molecules to be individually resolved. Thus, advantageously, the massive sequencing capacity of a single chip could be used to sequence large subsets of a genome across many individuals. DNA samples from control and affected patients may be run on the same array for example. Moreover, by tagging the source of each nucleic acid sample in a pool, there is no need to stringently measure and balance the absolute quantities of each DNA source mixed in a pool as is requisite in conventional pooling methodologies where an undetermined excess of individual input DNAs can result in an incorrect estimation of allele frequencies. By knowing the source of each DNA after pooling and analysis, allele frequencies can be determined with respect to the total number of pooled samples rather than the absolute quantity of DNA.

The utility of the invention is not, however, restricted to single molecule arrays. The method can also be applied to clustered arrays, and in particular clustered arrays generated by solid phase nucleic acid amplification, as will be apparent from the following description.

### Brief description of the drawings

The present invention may be more clearly understood from the following description and examples which are not intended to limit the invention but are by way of illustration, and with reference to the accompanying Figures wherein,

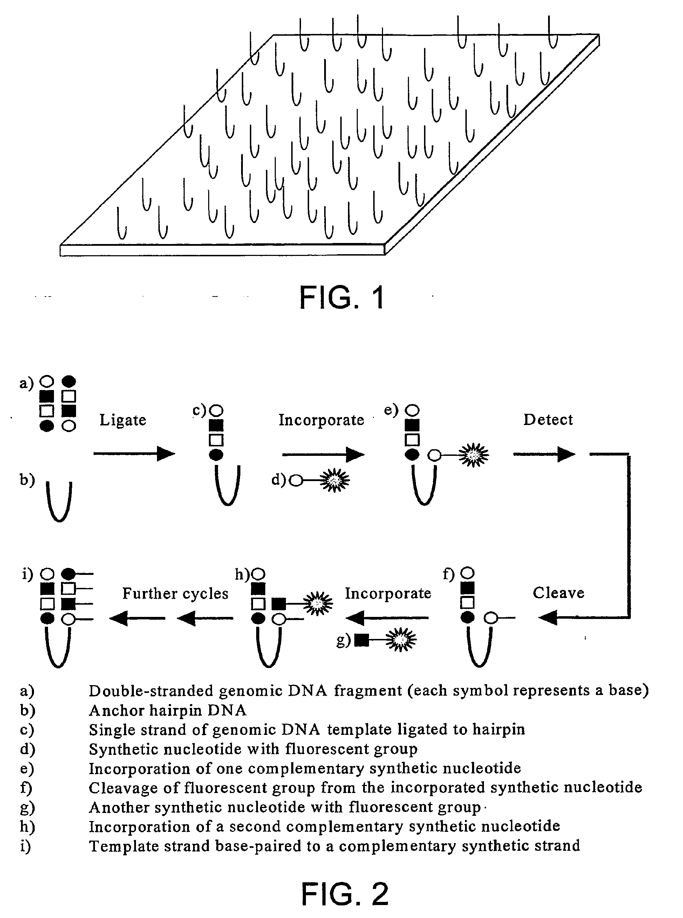

Figure 1 is a representational illustration of an array of hairpin-genomic DNA constructs immobilised on a glass slide. Key: a) a double-stranded genomic DNA fragment (each symbol represents a base), b) anchor hairpin DNA, c) single strand of genomic DNA template ligated to hairpin, d) synthetic nucleotide with fluorescent group, e) incorporation of one complementary synthetic nucleotide, f) cleavage of fluorescent group from the incorporated synthetic nucleotide, g) another synthetic nucleotide with fluorescent group, h) incorporation of a second complementary synthetic nucleotide and i) template strand base-paired to a complementary synthetic strand.

Figure 2 is a schematic illustration of multiple cycles of sequencing on a single molecule.

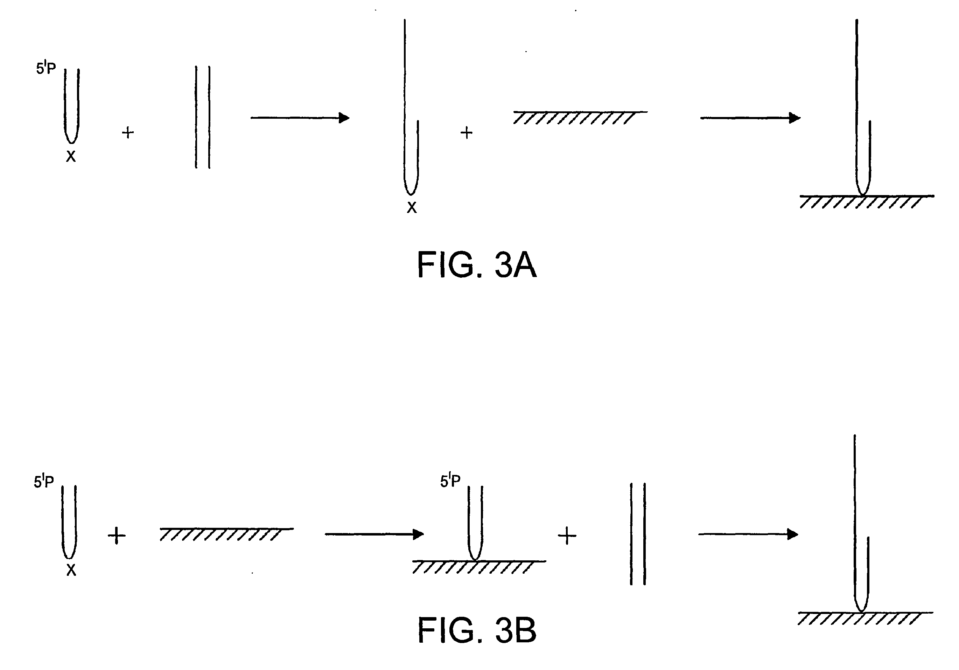

Figure 3a illustrates ligation of genomic DNA to a hairpin prior to immobilisation onto a surface. X denotes a functionality for coupling the hairpin to a surface.

Figure 3b illustrates ligation of genomic DNA to a hairpin already immobilised on a surface. X denotes a functionality for coupling the hairpin to a surface.

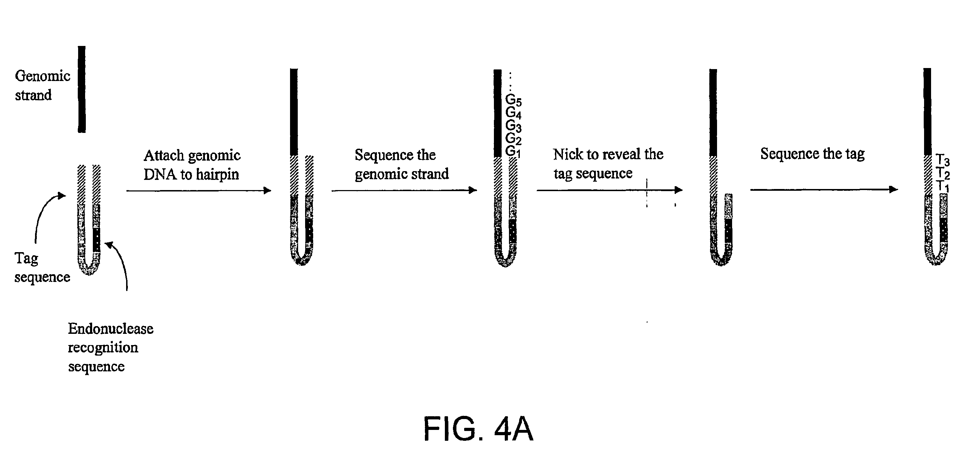

Figure 4a is a schematic illustration demonstrating sequencing of a tag after sequencing of the genomic DNA template and nicking to remove the sequencing strand. G₁G₂G₃G₄G₅... and T₁T₂T₃... refer to the first few bases of the synthetic strand complementary to the genomic DNA and the tag sequence, respectively.

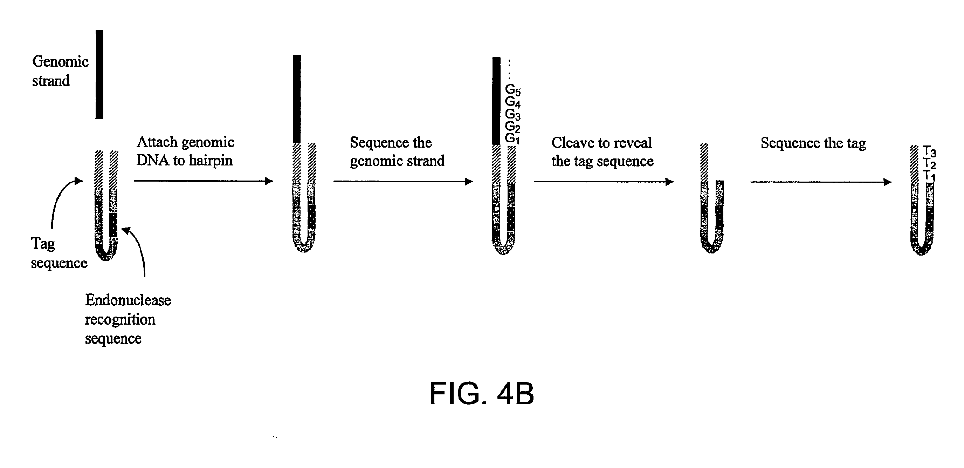

Figure 4b is a schematic illustration demonstrating sequencing of a tag after sequencing of the genomic DNA template and cleavage of both the sequencing strand and template strand to leave an overhang. G₁G₂G₃G₄G₅... and T₁T₂T₃... refer to the first few bases of the synthetic strand complementary to the genomic DNA and the tag sequence, respectively.

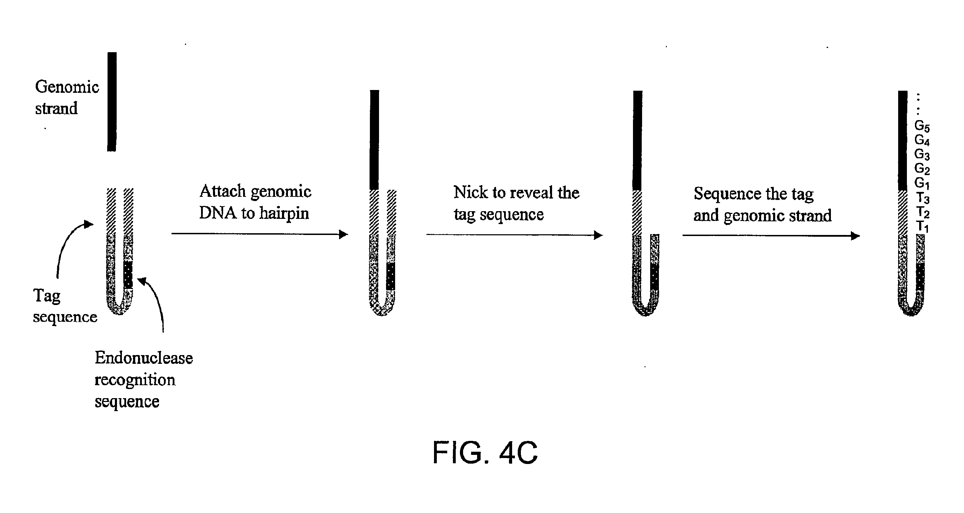

Figure 4c is a schematic illustration demonstrating nicking of the hairpin-genomic DNA construct prior to concomitant sequencing of the tag and genomic template. G₁G₂G₃G₄G₅... and T₁T₂T₃... refer to the first few bases of the synthetic strand complementary to the genomic DNA and the tag sequence, respectively.

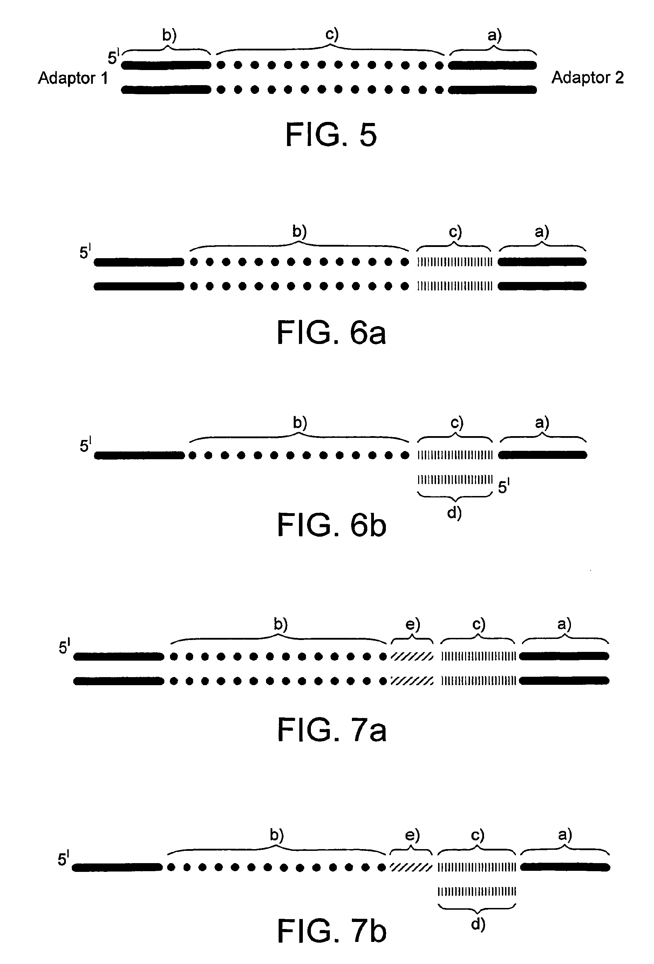

Figure 5 is a schematic illustration of a template nucleic acid construct used for production of clustered arrays of immobilised nucleic acid molecules via solid-phase amplification. The construct is a double-stranded polynucleotide molecule which comprises a nucleic acid fragment, which can be any nucleic acid fragment of interest, of known or unknown sequence, flanked by first and second adaptors (adaptor 1, adaptor 2) which respectively comprise amplification primer sequences b) and a).

Figure 6a is a schematic representation of a template nucleic acid construct which is a double-stranded polynucleotide comprising a nucleic acid fragment b) flanked by first and second adaptors. One of the adaptors comprises an amplification primer sequence a) and a sequencing primer binding sequence b). This construct can be used for production of clustered arrays of immobilised nucleic acid molecules via solid-phase amplification. Such clustered arrays will comprise immobilised polynucleotide molecules having the structure shown in Figure 6b.

Figure 6b is a schematic illustration of a polynucleotide molecule corresponding to one strand (the template strand) of the template construct shown in Figure 6a. A universal sequencing primer d) is shown hybridised to the complementary sequencing primer binding sequence.

Figure 7a is a schematic representation of a template nucleic acid construct according to the invention which is a double-stranded polynucleotide comprising a nucleic acid fragment b) flanked by first and second adaptors. One of the adaptors comprises an amplification primer sequence a), a sequencing primer binding sequence b) and a tag sequence e). This construct can be used for production of clustered arrays of immobilised nucleic acid molecules via solid-phase amplification. Such clustered arrays will comprise immobilised polynucleotide molecules having the structure shown in Figure 7b.

Figure 7b is a schematic illustration of a polynucleotide molecule corresponding to one strand of the template construct shown in Figure 7a. A universal sequencing primer d) is shown hybridised to the complementary sequencing primer binding sequence.

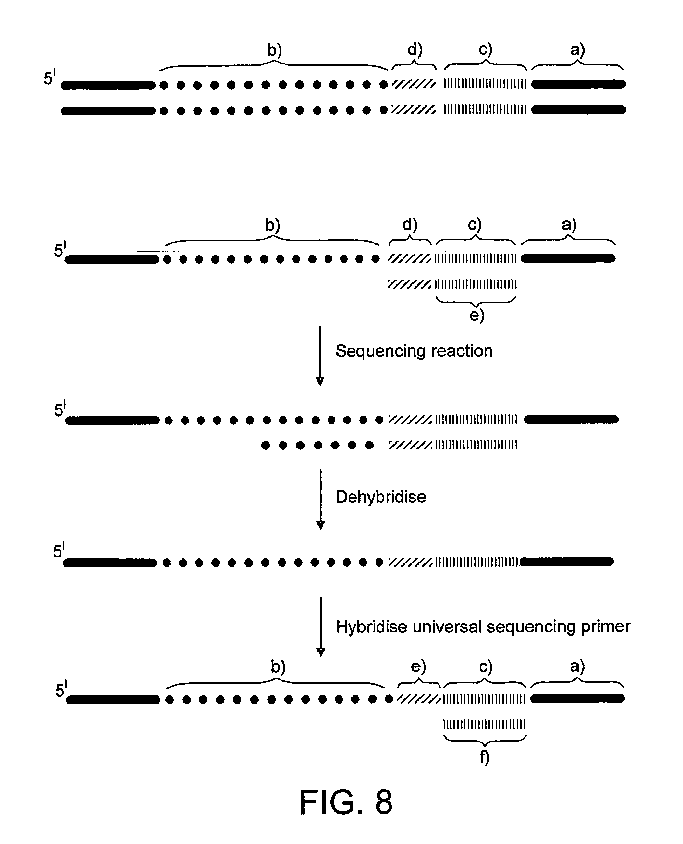

Figure 8 is a schematic illustration of a method of sequencing on a clustered array according to the invention.

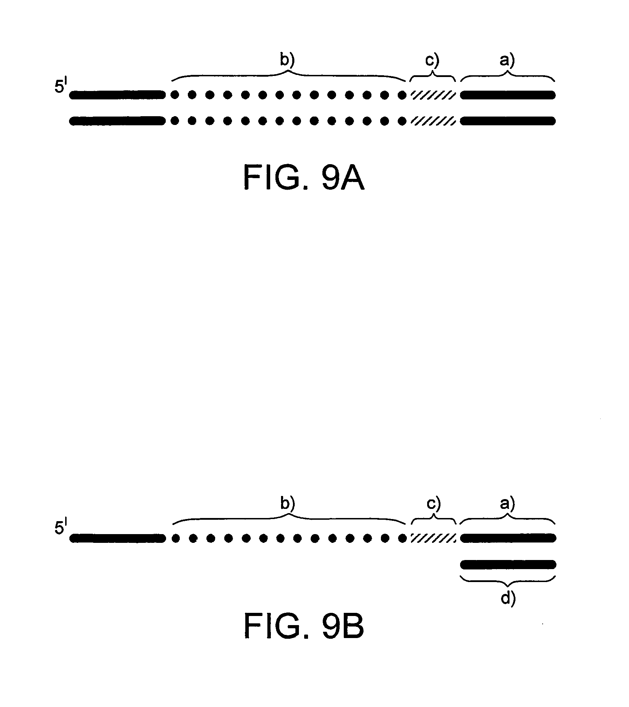

Figure 9a is a schematic representation of a template nucleic acid construct according to the invention which is a double-stranded polynucleotide comprising a nucleic acid fragment b) flanked by first and second adaptors. One of the adaptors comprises an amplification primer sequence a), and a tag sequence c). The amplification primer sequence also serves as a binding site for a universal sequencing primer. This construct can be used for production of clustered arrays of immobilised nucleic acid molecules via solid-phase amplification. Such clustered arrays will comprise immobilised polynucleotide molecules having the structure shown in Figure 9b.

Figure 9b is a schematic illustration of a polynucleotide molecule corresponding to one strand of the template construct shown in Figure 9a. A universal sequencing primer d) is shown hybridised to the complementary amplification primer (binding) sequence.

### Detailed description of specific embodiments

Presently, many sequencing technologies enable DNA from only a single source to be sequenced on an array while DNA from more than one source (*e.g.*, several different patients) may be pooled, fragmented and sequenced. During the subsequent analysis of the fragments, there is no way of discerning the origin of each fragment. The invention described herein enables this discernment and hence the sequencing of DNA from more than one source on an array and particularly an array of single molecules.

The present invention therefore comprises a sequencing methodology that can distinguish DNA or other target nucleic acid molecules from different sources on an array.

Suitable target nucleic acid molecules include genomic DNA. A specific embodiment of the method may involve fragmenting genomic DNA to less than 400 bp in length, preferably less than 100 bp, dephosphorylating the 5' ends, then coupling the DNA to the array via a capture moiety, such as a double-stranded nucleic acid anchor, for example a DNA hairpin oligonucleotide. While the present invention may be described with respect to genomic DNA and hairpin oligonucleotides, it will be apparent that other target nucleic acid molecules known to the skilled practitioner may also be employed.

The DNA molecules may be attached to the capture moiety for subsequent attachment to the array.

Therefore, in the case where the anchor comprises a hairpin oligonucleotide, the hairpin may contain a functionality at its looped end that allows it to be covalently coupled to a solid surface, for example, a glass slide. At its other end, the hairpin may comprise a 5' phosphate moiety and a 3' OH that enables it to be covalently coupled to only one of the two strands of a dephosphorylated genomic DNA fragment. The non-contiguous strand of the genomic DNA may then be removed by methods known to those skilled in the art. The coupled hairpin-genomic DNA construct is then attached to the solid surface (Figure 3A).

The array preferably consists of a surface with multiples of a nucleic acid construct, each construct preferably consisting of a universal double-stranded hairpin attached to a unique single-stranded genomic DNA fragment. The complementary (or 3') strand of the double-stranded hairpin that is not contiguous with the genomic DNA strand forms a primer for sequencing of the genomic DNA strand. Sequencing is performed by incorporating a fluorescently labelled nucleotide at the end of the primer that is complementary to the first base of the genomic DNA strand (Figure 2). The inclusion of a blocking functionality on the fluorescent nucleotide ensures that only one nucleotide is incorporated. Removal of the block and the fluorescence enables another single nucleotide incorporation complementary to the second base of the genomic DNA strand. The process is iterated and at each cycle the fluorescence of the incorporated nucleotide is recorded, preferably by a microscope and camera. The use of a different fluorophore for each of the four nucleotides, A, C, G and T enables the identity of the bases added during the sequencing reaction to be determine, and hence the sequence of the genomic DNA strand to be inferred by conventional base-pairing rules. The camera is capable of resolving and recording the fluorescence incorporated at many individual molecules simultaneously, preferably 1000 to 2000 molecules simultaneously from an image area of 0.01 mm². If repeated over a 10 cm² area, then sequence data from 10⁹ fragments of genomic DNA can be acquired. These sequenced fragments, preferably 20 to 30 bases in length, can be reassembled into their original genome contiguity by alignment to a reference sequence database.

DNA from more than one source can be sequenced on a sequencing array if the double stranded nucleic acid anchors are designed such that the end bases that immediately adjoin the genomic DNA strand form a variable tag sequence (Figures 4A, B, C). This tag sequence can be up to 100 nucleotides in length (base pairs if referring to double stranded molecules), preferably 1 to 10 nucleotides in length, most preferably 4, 5 or 6 nucleotides in length and comprises combinations of nucleotides. For example, in one embodiment, if six base-pairs are chosen to form the tag and a permutation of four different nucleotides used, then a total of 4096 nucleic acid anchors (e.g. hairpins), each with a unique 6 base tag can be made. This variable sequence tag may commence at the hairpin nucleic acid base immediately next to the genomic DNA strand or it may commence at a position more distal to the junction between the nucleic acid hairpin and the genomic DNA strand.

The double-stranded anchor may also contain a recognition sequence of an endonuclease, preferably a nicking endonuclease, capable of directing cleavage at a cleavage site immediately adjacent to, upstream of or within the complement of the tag sequence (Figure 4A, B, C). Following cleavage with the endonuclease, the tag sequence or part thereof is rendered single-stranded and suitable for further sequencing to determine the bases of the tag. Thus, the original source of the target nucleic acid fragment can be determined.

In one embodiment of the invention (Figure 4A), the genomic DNA strand is sequenced first, then the endonuclease, preferably a nicking endonuclease, is added to cleave and expose the tag sequence. In some cases of this embodiment (Figure 4B), an endonuclease can be used that cleaves in both strands of the tag generating a 5' overhang. The sequence of the tag is then determined in a second round of sequencing. In another embodiment (Figure 4C), the endonuclease is added to the coupled hairpin-genomic DNA construct prior to sequencing to generate a single-stranded portion of the construct that encompasses the single-stranded genomic DNA and a single-stranded tag sequence. In this format, the tag and genomic DNA are sequenced in the same round of sequencing cycles.

"Solid support", as used herein, refers to the material to which the polynucleotides molecules are attached. Suitable solid supports are available commercially, and will be apparent to the skilled person. The supports can be manufactured from materials such as glass, ceramics, silica and silicon. Supports with a gold surface may also be used. The supports usually comprise a flat (planar) surface, or at least a structure in which the polynucleotides to be interrogated are in approximately the same plane. Alternatively, the solid support can be non-planar, *e.g.*, a microbead. Any suitable size may be used. For example, the supports might be on the order of 1-10 cm in each direction.

The term "individually resolvable by optical microscopy" is used herein to indicate that, when visualised, it is possible to distinguish either at least one target polynucleotide on the array from its neighbouring polynucleotides using optical microscopy methods available in the art. Visualisation may be effected by the use of reporter labels, *e.g.*, fluorophores, the signal of which is individually resolved.

As used herein, the term "interrogate" means contacting one or more of the complementary copies of the target polynucleotides with another molecule, *e.g.*, a polymerase, a nucleoside triphosphate or a complementary nucleic acid sequence, wherein the physical interaction provides information regarding a characteristic of the arrayed target polynucleotide. The contacting can involve covalent or non-covalent interactions with the other molecule. As used herein, "information regarding a characteristic" means information regarding the sequence of one or more nucleotides in the target polynucleotide, the length of the target polynucleotide, the base composition of the target polynucleotide, the Tₘ of the target polynucleotide, the presence of a specific binding site for a polypeptide or other molecule, the presence of an adduct or modified nucleotide, or the three-dimensional structure of the polynucleotide.

As aforementioned, the target molecule may be capable of being attached to the solid support by virtue of a chemical or other functionality thereon that can interact with a complementary capture moiety to effect attachment to the surface of the support. The capture moiety may comprise a sequence of nucleotides that is capable of hybridising with a complementary sequence on the target molecule. The capture moiety may itself be present on the surface of the support and thus may itself include means for attachment to the surface of the support. In this regard, the target nucleic acid may include a further adaptor molecule that can hybridise to the sequence of nucleotides on the capture moiety, which adaptor molecule or sequence may be positioned at the 3' end of the nucleic acid. Thus, advantageously, the capture moiety may itself act as a primer for surface dependent amplification of the target nucleic acid.

In a preferred embodiment the capture moiety comprises a hairpin oligonucleotide. In one embodiment, "hairpin oligonucleotide" means a single-stranded nucleic acid molecule which is capable of forming a hairpin, that is, a nucleic acid molecule whose sequence contains a region of internal self-complementarity enabling the formation of an intramolecular duplex or self-hybrid. "Region of self-complementarity" refers to self-complementarity over a region of 4 to 100 base pairs. When not self-hybridized, the hairpin oligonucleotide can be 8 to 200 base pairs, preferably 10 to 30 base pairs in length. By saying that the hairpin oligonucleotide is a "self-hybrid", or that the hairpin oligonucleotide has "self-hybridized", means that the hairpin oligonucleotide has been exposed to conditions that allow its regions of self-complementarity to hybridize to each other, forming a double-stranded nucleic acid molecule with a loop structure at one end and exposed 3' and 5' ends at the other.

In another embodiment, the hairpin oligonucleotide is synthesized in a contiguous fashion but is not made up entirely of DNA, rather the ends of the molecule comprise DNA bases that are self-complementary and can thus form an intramolecular duplex, while the middle of the molecule includes one or more non-nucleic acid molecules. An example of such a hairpin nucleic acid molecule would be Nu-Nu-Nu-Nu-Nu-LM-Nc-Nc-Nc-Nc-Nc, where "Nu" is a particular nucleotide, "Nc" is the nucleotide complementary to Nu, and "LM" is the linker moiety linking the two strands, e.g., hexaethylene glycol (HEG) or polyethylene glycol (PEG). The non-nucleic acid molecule(s) can be linker moieties for linking the two nucleic acids together (the two nucleic acid halves of the overall hairpin nucleic acid molecule), and can also be used to attach the overall hairpin nucleic acid molecule to the substrate. Alternatively, the non-nucleic acid molecule(s) can be intermediate molecules which are in turn attached to linker moieties used for attaching the overall hairpin nucleic acid to the solid substrate.

In another embodiment, the hairpin oligonucleotide may be composed of two separate but complementary nucleic acid strands that are hybridized together to form an intermolecular duplex, and are then covalently linked together. The linkage can be accomplished by chemical crosslinking of the two strands, attaching both strands to one or more intercalators or chemical crosslinkers, etc.

In a preferred embodiment of the invention, the hairpin molecule includes a 3' overhang which is taken to mean that at the 3' end of the hairpin molecule, there is provided a sequence of nucleotides which do not hybridise to a complementary region.

In this embodiment the 3' end of the hairpin may include a 3' block. An adaptor molecule on said target nucleic acid molecule is preferably complementary to a sequence on the 3' end of the hairpin oligonucleotide. Therefore, once the target nucleic acid molecule including said 3' adaptor molecule is brought into contact with said hairpin molecule, the 3' adaptor molecule on the target molecule will hybridise to its complementary sequence on the 3' overhanging sequence of the hairpin. The hairpin oligonucleotide also preferably includes a phosphate moiety at the 5' terminus thereof so that the 3' end of the target nucleic acid molecule can be ligated thereto in the presence of an appropriate ligase enzyme. Accordingly, in this embodiment the hairpin oligonucleotide must be designed such that upon hybridisation of the 3' region of the target molecule (with 3' adaptor) to its complementary sequence on the 3' of the hairpin, the phosphate moiety on the 5' end is sufficiently proximal to the 3' end of the target nucleic acid molecule so as to be capable of undergoing a ligation reaction. Once the stabilised ligation product is generated, the sequence at the 3' end of the hairpin complementary to that of the 3' end of the target can serve as a primer for a subsequent polymerase based sequencing reaction.

Immobilisation of the hairpin oligonucleotides may be by specific covalent or non-covalent interactions. In the present invention, biotin may be used to immobilise the hairpin oligonucleotides to a streptavidin coated solid support. Immobilisation may also be carried out using covalent means such as amino or thiol oligonucleotides onto activated carboxy, maleimide or other suitably reactive surfaces.

Double stranded anchors, including hairpins, and other capture moieties comprising or consisting of polynucleotide molecules may include natural and/or non-natural bases and also natural and/or non-natural backbone linkages.

The target nucleic acid molecule used in accordance with the invention may typically be DNA or RNA, although nucleic acid mimics, e.g., PNA or 2'-O-methyl-RNA, are within the scope of the invention.

A first step in the fabrication of the arrays will usually be to functionalise the surface of the solid support, making it suitable for attachment of the molecules/polynucleotides. Biotinylated albumins (BSA) can form a stable attachment of biotin groups by physisorption of the protein onto surfaces. Covalent modification can be performed using silanes, which have been used to attach molecules to a solid support, usually a glass slide. Biotin molecules can be attached to surfaces using appropriately reactive species such as biotin-PEG-succinimidyl ester which reacts with an amino surface. The molecules can then be brought into contact with the functionalised solid support, to form the arrays.

In an alternative embodiment, the support surface may be treated with different functional groups, one of which is to react specifically with different target molecules. Controlling the concentration of each functional group provides a convenient way to control the densities of the hairpin molecules/target nucleic acid.

Suitable functional groups will be apparent to the skilled person. For example, suitable groups include: amines, acids, esters, activated acids, acid halides, alcohols, thiols, disulfides, olefins, dienes, halogenated electrophiles, thiophosphates and phosphorothioates.

In one embodiment, the unreactive silanes may be of the type RnSiX(4-n) (where R is an inert moiety that is displayed on the surface of the solid support, n is an integer from 1-4 and X is or comprises a reactive leaving group, such as a halide (e.g. Cl, Br) or alkoxide e.g. (1-6 alkoxide). Such modified surfaces may be created by reactions with silanes, such as tetraethoxysilane, triethoxymethylsilane, diethoxydimethylsilane or glycidoxypropyltriethoxysilane, although many other suitable examples will be apparent to the skilled person.

The target nucleic acid molecules may be immobilised onto the surface of the solid support to form a single molecule array (SMA) in which the target nucleic acid molecules are capable of being resolved by optical means. This means that, within the resolvable area of the particular imaging device used, there must be one or more distinct signals, each representing one polynucleotide. Thus, each molecule is individually resolvable and detectable as a single molecule fluorescent point, and fluorescence from said single molecule fluorescent point also exhibits single step photobleaching.

Clusters of substantially identical molecules do not exhibit single point photobleaching under standard operating conditions used to detect/analyze molecules on arrays. The intensity of a single molecule fluorescence spot is constant for an anticipated period of time after which it disappears in a single step. In contrast, the intensity of a fluorescence spot comprised of two or more molecules, for example, disappears in two or more distinct and observable steps, as appropriate. The intensity of a fluorescence spot arising from a cluster consisting of thousands of similar molecules, such as those present on the arrays consisting of thousands of similar molecules at any given point, for example, would disappear in a pattern consistent with an exponential decay. The exponential decay pattern reflects the progressive loss of fluorescence by molecules present in the cluster and reveals that, over time, fewer and fewer molecules in the spot retain their fluorescence.

Typically, the polynucleotides on a single molecule array are resolved using a single molecule fluorescence microscope equipped with a sensitive detector, e.g., a charge-coupled device (CCD). Each polynucleotide of the array may be imaged simultaneously or, by scanning the array, a fast sequential analysis can be performed. While the density of the polynucleotides is not critical, it must be such as to render the polynucleotides individually resolvable as hereinbefore described. Preferably, however, the polynucleotides are provided in the range of 10⁶ to 10⁹ polynucleotides per cm² and even more preferably 10⁷ to 10⁸ /cm² or one molecule is provided per 250 nm² or per 62500 nm².

Once formed the arrayed polynucleotides may be used in procedures to determine the sequence of the target nucleic acid molecule. In particular, the single molecule arrays may be used in conventional assays which rely on the detection of fluorescent labels to obtain information on the arrayed polynucleotides. The arrays are particularly suitable for use in multi-step assays where the loss of synchronisation in the steps was previously regarded as a limitation to the use of arrays. The arrays may be used in conventional techniques for obtaining genetic sequence information. Many of these techniques rely on the stepwise identification of suitably labelled nucleotides, referred to in US-A-5654413 as "single base" sequencing methods or "sequencing-by-synthesis".

In an embodiment of the invention, the sequence(s) of the target polynucleotide may be determined in a similar manner to that described in US-A-5654413, by detecting the incorporation of nucleotides into the nascent strand through the detection of a fluorescent label attached to the incorporated nucleotide. In the present invention, the primer is located on the 3' end of the hairpin oligonucleotide following ligation of the 3' end of the target nucleic acid molecule to the 5' end of the hairpin. The nascent chain may then be extended in a stepwise manner by the polymerase reaction. Each of the different nucleotides (A, T, G and C) incorporates a unique fluorophore and a block at the 3' position on the nucleotide acts as a blocking group to prevent uncontrolled polymerisation. The polymerase enzyme incorporates a nucleotide into the nascent chain complementary to the target, and the blocking group prevents further incorporation of nucleotides. The array surface is then cleared of unincorporated nucleotides and each incorporated nucleotide is "read" optically by a charge-coupled device using laser excitation and filters. The 3' -blocking group is then removed (deprotected), to expose the nascent chain for further nucleotide incorporation.

US Patent No. 5,302,509 also discloses another method to sequence polynucleotides immobilised on a solid support. The method relies on the incorporation of fluorescently-labelled, 3'-blocked bases A, G, C and T to the immobilised polynucleotide, in the presence of DNA polymerase. The polymerase incorporates a base complementary to the target polynucleotide, but is prevented from further addition by the 3'-blocking group. The label of the incorporated base can then be determined and the blocking group removed by chemical cleavage to allow further polymerisation to occur.

Other suitable sequencing procedures will be apparent to the skilled person. In particular, the sequencing method may rely on the degradation of the arrayed polynucleotides, the degradation products being characterised to determine the sequence.

An example of a suitable degradation technique is disclosed in WO-A- 95/20053, whereby bases on a polynucleotide are removed sequentially, a predetermined number at a time, through the use of labelled adaptors specific for the bases, and a defined exonuclease cleavage.

However a consequence of sequencing using non-destructive methods is that it is possible to form a spatially addressable array for further characterisation studies, and therefore non-destructive sequencing may be preferred. In this context, the term "spatially addressable" is used herein to describe how different single nucleic acid molecules may be identified on the basis of their position on an array.

In the case that the target nucleic acid molecules are generated by restriction digest of genomic DNA, the recognition sequence of the restriction or other nuclease enzyme will provide 4, 6, 8 bases or more of known sequence (dependent on the enzyme). However, as aforementioned, adaptor molecules of known sequence can be added to the 3' ends thereof. Further sequencing of between 10 and 20 bases on the array should provide sufficient overall sequence information to place that stretch of DNA into unique context with a total human genome sequence, thus enabling the sequence information to be used for genotyping and more specifically single nucleotide polymorphism (SNP) scoring.

Thus the arrays of this invention may be incorporated into, for example, a sequencing machine or genetic analysis machine.

The polynucleotides immobilised onto the surface of a solid support to form a single molecule array should be capable of being resolved by optical means. This means that, within the resolvable area of the particular imaging device used, there must be one or more distinct signals, each representing one single molecule. Typically, the polynucleotides of the array are resolved using a single molecule fluorescence microscope equipped with a sensitive detector, e.g., a charge-coupled device (CCD). Each polynucleotide of the array may be imaged simultaneously or, by scanning the array, a fast sequential analysis can be performed.

The extent of separation between the individual polynucleotides on a single molecule array will be determined, in part, by the particular technique used to resolve the polynucleotides. Apparatus used to image molecular arrays are known to those skilled in the art. For example, a confocal scanning microscope may be used to scan the surface of the array with a laser to image directly a fluorophore incorporated on the individual polynucleotide by fluorescence. Alternatively, a sensitive 2-D detector, such as a charge-coupled device, can be used to provide a 2-D image representing the individual polynucleotides on the array.

"Resolving" single molecules on the array with a 2-D detector can be done if, at 100 x magnification, adjacent molecules on the array are separated by a distance of approximately at least 250 nm, preferably at lest 300 nm and more preferably at least 350 nm. It will be appreciated that these distances are dependent on magnification, and that other values can be determined accordingly, by one of ordinary skill in the art.

Other techniques such as scanning near-field optical microscopy (SNOM) are available which are capable of greater optical resolution, thereby permitting more dense arrays to be used. For example, using SNOM, adjacent polynucleotides may be separated by a distance of less than 100 nm, e.g., 10 nm. For a description of scanning near-field optical microscopy, see Moyer et al., Laser Focus World (1993) 29 (10) .

An additional technique that may be used is surface-specific total internal reflection fluorescence microscopy (TIRFM); see, for example, Vale et al., Nature (1996) 380:451-453). Using this technique, it is possible to achieve wide-field imaging (up to 100 µm x 100 µm) with single molecule sensitivity. This may allow arrays of greater than 10⁷ resolvable polynucleotides per cm² to be used.

Additionally, the techniques of scanning tunnelling microscopy (Binnig et al., Helvetica Physica Acta (1982) 55:726-735) and atomic force microscopy (Hansma et al., Ann. Rev. Biophys. Biomol. Struct. (1994) 23:115-139) are suitable for imaging the arrays of the present invention. Other devices which do not rely on microscopy may also be used, provided that they are capable of imaging within discrete areas on a solid support.

The utility of the invention is not limited to sequencing target molecules on single molecule arrays. In addition, the methods of the invention can also be applied to clustered arrays, and in particular clustered arrays formed by amplification of a target nucleic acid molecule on a solid support.

Therefore, in one embodiment of the invention the array is a clustered array. In a preferred embodiment the clustered array will be formed by solid-phase amplification. In this embodiment, the individual nucleic acid molecules immobilised on the array which serve as templates for subsequent sequencing will be amplification products of the solid-phase amplification reaction.

The formation of clustered arrays comprised of pluralities of immobilised nucleic acid molecules (also referred to as nucleic acid colonies) by nucleic acid amplification on a solid support is described in general terms in, for example, WO 98/44151 and WO 00/18957. The amplification techniques described therein may be adapted in order to prepare clustered arrays incorporating sequence tags according to the invention.

A key step in the generation of clustered arrays by amplification is the attachment of known adaptor sequences to the ends of target nucleic acid molecules to be amplified (e.g. random fragments of human genomic DNA) that enable amplification of these molecules on a solid support to form clusters. The adaptors are typically short oligonucleotides that may be synthesised by conventional means. The adaptors may be attached to the 5' and 3' ends of target nucleic acid fragments by a variety of means (e.g. subcloning, ligation. etc). More specifically, two different adaptor sequences are attached to a target nucleic acid molecule to be amplified such that one adaptor is attached at one end of the target nucleic acid sequence in the target molecule and another adaptor is attached at the other end of the target nucleic acid molecule. The resultant construct comprising a target nucleic acid sequence flanked by adaptors may be referred to herein as a "template nucleic acid construct".

The adaptors contain sequences which permit nucleic acid amplification using amplification primer molecules immobilised on a solid surface. These sequences in the adaptors may be referred to herein as "amplification primer sequences". In order to act as a template for nucleic acid amplification, a single strand of the template construct must contain a sequence which is complementary to a first amplification primer molecule (such that the first primer molecule can bind and prime synthesis of a complementary strand) and a sequence which corresponds to the sequence of a second amplification primer molecule (such that the primer molecule can bind to the complementary strand). The sequences in the adaptors which permit hybridisation to primer molecules will typically be around 20-25 nucleotides in length, although the invention is not limited to sequences of this length. The term "hybrisidation" encompasses sequence-specific binding between primer and template. During the amplification reaction, binding of an immobilised primer molecule to its cognate sequence in the template can occur under typical conditions used for primer-template annealing in standard PCR.

The precise identity of the amplification primer sequences, and hence the sequence of the cognate amplification primer molecules, is generally not material to the invention, as long as the primer molecules are able to interact with the amplification sequences in order to direct PCR amplification. The criteria for design of PCR primers are generally well known to those of ordinary skill in the art.

The amplification primer molecules are oligonucleotide molecules which may comprise a functionality enabling attachment to a solid support. Suitable primers can be synthesised using standard synthetic techniques well known in the art. Attachment can be by any suitable attachment means or attachment known in the art, including any attachment means described herein in connection with any other aspect of the invention. Once immobilised, they serve as amplification primers for nucleic acid amplification on the solid support (illustrated schematically in Figure 5).

In embodiments based on the formation of clustered arrays the "capture moiety" can be considered to be the functional group which is used for immobilisation of the amplification primers to the array. Molecules comprising the target nucleic acid sequence together with a tag characteristic of the source of the target nucleic acid sequence are thus immobilised on the array via such capture moieties as a result of solid-phase amplification using the immobilised capture primers. Solid-phase amplification results in the formation of amplification products comprising the target nucleic acid sequence and the tag sequence immobilised on the array.

In one embodiment of the invention solid phase amplification may be carried out as follows: both amplification primers are first immobilised on the solid support by an suitable attachment chemistry. Following attachment of the primers the solid support is contacted with the template to be amplified under conditions which permit hybridisation between the template and the immobilised primers. The template is generally added in free solution and suitable hybridisation conditions will be apparent to the skilled reader. Typically hybridisation conditions are, for example, 5xSSC at 40°C, following an initial denaturation step. Solid-phase amplification can then proceed, the first step of the amplification being a primer extension step in which nucleotides are added to the 3' end of the immobilised primer hybridised to the template to produce a fully extended complementary strand. This complementary strand will thus include at its 3' end a sequence which is capable of binding to the second primer molecule immobilised on the solid support. Further rounds of amplification (analogous to a standard PCR reaction) lead to the formation of clusters or colonies of template molecules bound to the solid support.

In an alternative embodiment of the invention the amplification primers and template constructs may be mixed and then immobilised on the solid support in a single attachment step. In this embodiment the amplification reaction is substantially similar to that described in WO 98/44151 and WO 00/18957.

DNA amplification on solid supports is a procedure well documented in the literature. A wide range of support types (e.g. microarrays (Huber M. et al. (2001) Anal. Biochem. 299(1), 24-30; Rovera G. (2001) US patent 6221635 B1 20010424), glass beads (Adessi C. et al. (2000) Nucl. Acids Res. 28(20), e87; Andreadis J. D. et al. (2000) Nucl. Acids Res. 28(2), e5), agarose (Stamm S. et al. (1991) Nucl. Acids Res. 19(6), 1350) or polyacrylamide (Shapero M. H. et al. (2001) Genome Res. 11,1926-1934; Mitra, R. D. et al. (1999) Nucl. Acids Res. 27(24), e34)) and attachment chemistries (e.g. 5'-thiol oligo on aminosilane slides via heterofunctional crosslinker (Adessi C. et al. (2000) Nucl. Acids Res. 28(20), e87; Andreadis J. D. et al. (2000) Nucl. Acids Res. 28(2), e5), EDC chemistry on NucleoLink^{™} surface (Sjoroos M. et al. (2001) Clin. Chem. 47(3), 498-504) or amino silane (Adessi C. et al. (2000) Nucl. Acids Res. 28(20), e87), radical polymerization (Shapero M. H. et al. (2001) Genome Res. 11,1926-1934; Mitra, R. D.et al. (1999) Nucl. Acids Res. 27(24), e34) ) have been described. PCR on polyacrylamide coated glass slides (Shapero *et al*., ibid) or beads (Mitra *et al*., ibid) has also been reported.

One or both of the adaptors flanking the target nucleic acid sequence may also include an additional sequence complementary to a sequencing primer. In the target molecules immobilised on the array (i.e. the products of solid-phase amplification) this additional sequence will be positioned between the amplification primer sequence and the target nucleic acid sequence, such that when a sequencing primer is hybridised to the additional sequence the next base after that nucleotide base-paired the 3' end of the sequencing primer is the first base of the target nucleic acid molecule. The additional sequence is preferably included in the adaptor which is located distal from the solid support following amplification to generate clusters of immobilised molecules. A suitable template construct is illustrated in Figure 6a and amplified strands in Figure 6b, assuming attachment to the support via the 5' end. The additional sequence is herein referred to as the "sequencing primer binding sequence". The hybridising sequencing primer may also be referred to as the "universal sequencing primer" (illustrated schematically in Figure 6b). Binding of the sequencing primer to its cognate sequence in the adaptor provides an initiation point for sequencing of polynucleotide molecules on the clustered array.

A tag of additional bases can be included in the adaptor between the sequencing primer binding sequence and the target nucleic acid molecule to generate an encoded tag sequence in the immobilised molecules on the array (template construct illustrated schematically in Figure 7a and amplified strands in Figure 7b). The tag sequence is again an additional sequence added to the target nucleic acid sequence, typically to provide a marker of the source of the target nucleic acid sequence. Preferred features of the tag are as described for other embodiments of the invention.

In the embodiment illustrated in Figure 7, the first bases identified in a sequencing reaction initiated at a sequencing primer binding to the sequencing primer binding sequence will be the bases that form the tag; the next bases identified will be the first bases of the attached target nucleic acid sequence (illustrated schematically in Figure 7b). Thus, the adaptor will consist of the following order of constituents: the amplification primer sequence, the sequencing primer binding sequence, then the encoded tag sequence; the complete construct is herein referred to as an "encoded tag adaptor". This entire construct can be synthesised and added to target nucleic acid molecules/fragments using standard molecular biology techniques, such that the tag is positioned immediately adjacent to the target nucleic acid sequence.

The nucleic acid tag sequence may be included in either one or both of the adaptors present in the template nucleic acid constructs used in the construction of the arrays by solid-phase amplification. It will be appreciated that any given solid-phase amplification will result in the formation of two types of immobilised single-stranded amplification products which are complementary to each other. Following amplification one of the amplified strands may be removed from the array, for example by selective cleavage at a predetermined cleavage site in one of the adaptors, leaving only a single type of template strand on the array. The amplified template strands left on the array must include a target nucleic acid sequence and a nucleic acid tag sequence, plus a binding sequence for a sequencing primer in order to prime sequencing-by-synthesis of the template. Such strands are immobilised via their 5' ends, leaving the 3' end of the molecule free to act as a template for sequencing.

Many encoded tag adaptors can be designed, each with a unique tag sequence. A unique encoded tag adaptor can be attached to each of several sets of target nucleic acid fragments and the sets then pooled. When the resultant constructs are used as templates for the generation of a clustered array, all clusters derived from a given set of nucleic acid fragments will have the same tag sequence, which differs from the tag sequence in clusters derived from a different set of nucleic acid fragments. All clusters may include the same "sequencing primer" binding sequence, such that a universal sequencing primer can be used to sequence all the clusters from all sets of nucleic acid fragments. During a sequencing reaction on a clustered array, the tag sequence will be sequenced first, then the attached nucleic acid fragment. By this means the set of nucleic.acid fragments from which an individual nucleic acid fragment on the array originates can be identified following sequencing of a portion of the nucleic acid fragment and its associated tag.

In another embodiment of this invention, a sequencing primer can be designed with additional bases such that it hybridises to the sequencing primer binding sequence and the sequence of the tag. Such a primer may be herein referred to as a "tagged sequencing primer" (illustrated schematically in Figure 8) as opposed to a "universal" sequencing primer. For each unique encoded tag adaptor, a tagged sequencing primer can be designed that hybridises to the sequencing primer binding sequence and the sequence of the tag. During the sequencing reaction on a clustered array, a plurality of tagged sequencing primers are hybridised under stringent conditions to the array such that every tagged sequencing primer hybridises to its complementary sequencing primer binding sequence. In this instance, the first bases sequenced in a sequencing reaction are the first bases of the target nucleic acid fragment. Following a completed sequencing reaction, the tagged sequencing primer can be dehybridised and removed (i.e. by denaturation under standard conditions). A subsequent hybridisation of a universal sequencing primer followed by further sequencing reactions will identify the sequence of the tag.

The embodiment outlined in the preceding paragraph is illustrated schematically in Figure 8. i) illustrates a template nucleic acid construct comprising a nucleic acid fragment b) flanked by first and second adaptors. One of the adaptors comprises an amplification primer sequence a), a sequencing primer binding sequence b) and a tag sequence d). The template construct shown in i) can be used for production of clustered arrays of immobilised nucleic acid molecules via solid-phase amplification. Such clustered arrays will comprise immobilised single stranded polynucleotide molecules having the structure represented schematically in part ii). Part ii) shows a tagged sequencing primer hybridised to the complementary sequencing primer binding sequence and tag sequence. The primer provides an initiation point for a first sequencing reaction to determine the sequence of a portion of the nucleic acid fragment. In part iii) the extended primer produced in this first sequencing reaction is dehybridised (under standard denaturing conditions) to regenerate the template strand shown in part iv). The template strand may then be hybridised to a universal sequencing primer which is complementary to the sequencing primer binding sequence but not the tag sequence, as shown in part v). This universal primer provides an initiation point for sequencing of the tag sequence.

In a still further embodiment of this invention (schematically illustrated in Figure 9) the "sequencing primer binding sequence" can be provided by an amplification primer sequence in one of the adaptors, which thus performs a dual function of: (i) enabling cluster generation by virtue of its complementarity to amplification primers immobilised on the solid support, and (ii) enabling hybridisation of a universal sequencing primer. In this embodiment the encoded tag adaptor will consist of the following order of constituents: the amplification primer sequence, then the encoded tag (Figure 9).

The invention will be further understood with reference to the following experimental example.

### Example.

Two hairpins with the following sequence were synthesized by a commercial source: 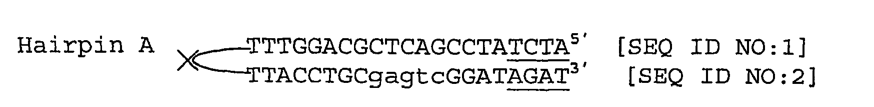
Hairpin A TTTGGACGCTCAGCCTATCTA5' [SEQ ID NO:1]
TTACCTGCgagtcGGATAGAT3' [SEQ ID NO:2]
 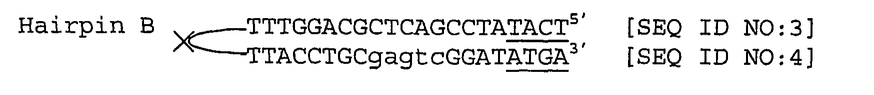
Hairpin B TTTGGACGCTCAGCCTATACT5' [SEQ ID NO:3]
TTACCTGCgagtcGGATATGA3' [SEQ ID NO:4]
 (sequence tags are underlined; X denotes a functionality for attaching the hairpin to a surface; bases in lower-case indicate a recognition site for N.BstNBI nicking endonuclease). Each hairpin was synthesised with a 5' phosphate.

Two double-stranded DNA template molecules were also synthesized as follows:
- Template 1: 5'TCTTGGAGTGGTGAATC [SEQ ID NO:5]
3' AGAACCTCACCACTTAGGC [SEQ ID NO:6]
- Template 2: 5' CGCTTCGTTAATACAGA [SEQ ID NO:7]
3' GCGAAGCAATTATGTCTAC [SEQ ID NO:8]

Each double-stranded template is blunt at one end and overhanging at the other; this ensures that only one end, the blunt end, joins with the blunt end of a hairpin.

A twenty microlitre reaction was prepared containing 10 pmoles of a DNA hairpin A, 10 pmoles of a double-stranded template 1, and several thousand units of a DNA ligase enzyme. A second reaction was prepared containing 10 pmoles of a DNA hairpin B, 10 pmoles of a double-stranded template 2, and several thousand units of a DNA ligase enzyme. The reactions were incubated at room temperature for 30 minutes, then purified by phenol/chloroform extraction upon completion. The action of the ligase enzyme fuses the hairpin and the double-stranded oligonucleotide at their blunt ends only, and because only the 5' end of the hairpin carries a phosphate group, the reaction results in joining one strand to the hairpin - the longer strand, as follows: 
TTTGGACGCTCAGCCTATCTA-AGAACCTCACCACTTAGGC
TTACCTGCgagtcGGAT▲AGAT TCTTGGAGTGGTGAATC
 
TTTGGACGCTCAGCCTATACT-GCGAAGCAATTATGTCTAC
TTACCTGCgagtcGGAT▲ATGA CGCTTCGTTAATACAGA
 (_{▲} indicates the nicking position of N.BstNBI; the hyphen indicates a chemical bond between the hairpin and the template DNA)

The purified reaction, resuspended in 10 µl of H₂O, were pooled together, then subjected to a nicking reaction at 55°C for 30 minutes with N.BstNBI (5 Units; New England Biolabs, Inc., Beverly, Massachusetts, USA), which nicks the extended DNA between the fourth and fifth base downstream of its recognition site and immediately before the tag sequence. The reaction was performed in the buffer recommended by the supplier of the enzyme. The pooled DNAs were then purified by phenol/chloroform extraction, coupled to a surface and subject to sequencing by single-molecule array sequencing protocols as described in WO 00/06770. The first four cycles of sequencing determine the identity of the hairpin whereas the subsequent cycles determine the sequence of the template DNA.

While this invention has been particularly shown and described with references to preferred embodiments, it will be understood by those skilled in the art that various changes in form and details may be made without departing from the scope of the invention encompassed by the claims.

## Claims

1. A method of sequencing and distinguishing between nucleic acid sequences on a single array, which sequences originate from different sources, which method comprises the steps of,
a) ligating target nucleic acid sequences comprising DNA polynucleotides from a first source to double-stranded nucleic acid anchoring molecules thereby producing tagged target nucleic acid sequences, wherein said double-stranded nucleic acid anchoring molecules comprise a nucleic acid sequence tag characteristic of the target nucleic acid sequence source and a functionality capable of effecting immobilisation to said array, and wherein the 3' end of each strand of said DNA polynucleotide is ligated to the 5' end of one strand of said double-stranded anchoring molecule, which 5' end is not used for anchoring;
b) repeating step a) for target nucleic acid sequences from second and subsequent sources using second and subsequent double-stranded nucleic acid anchoring molecules having characteristic nucleic acid sequence tags for each of said sources;
c) immobilising tagged target nucleic acid sequences from different sources to said array via the functionality capable of effecting immobilisation, thereby producing immobilised molecules, each immobilised molecule comprising a target nucleic acid sequence and a nucleic acid sequence tag characteristic of the target nucleic acid sequence source wherein said nucleic acid sequences on said array are disposed at a density such that they are capable of individual resolution using optical microscopy; and
d) sequencing said immobilised individually resolvable molecules by addition of nucleotides to a 3' hydroxyl group on the double-stranded anchor whereupon said sequencing identifies a sequence of each of the target nucleic acid molecules and the sequence of the tag to identify the corresponding source of the target nucleic acid molecule.

2. A method according to claim 1 wherein said double-stranded nucleic acid anchoring molecule is a hairpin oligonucleotide and said DNA polynucleotide is ligated to the 5' end of said hairpin nucleotide.

3. A method according to claim 1 or claim 2 wherein said double stranded anchoring molecule or hairpin comprises a 5' overhanging sequence and the nucleic acid sequence tag is located on said overhanging sequence.

4. A method according to claim 2 wherein said hairpin oligonucleotide comprises a single stranded nucleic acid sequence which contains a region of internal self complementarity, said region being capable of forming a intramolecular duplex.

5. A method according to claim 4 wherein said hairpin oligonucleotide comprises said nucleic acid sequence tag characteristic of said target nucleic acid sequence source positioned immediately adjacent the 5' end of said hairpin and the complement of said nucleic acid sequence tag positioned immediately adjacent the 3' end of said hairpin.

6. A method according to claim 5 wherein said target nucleic acid is ligated to said hairpin oligonucleotide by removing any phosphate groups from 5' and 3' ends of said target nucleic acid whilst providing a single phosphate group at the 5' end of said hairpin oligonucleotide, and incubating said target nucleic acid and hairpin oligonucleotide in the presence of a ligation reagent, whereby a 3' end of the target nucleic acid is ligated to the 5' end of said hairpin oligonucleotide.

7. A method according to any of claims 3 to 6 wherein said overhanging sequence is generated in a cleavage step comprising cleavage of the 3' strand of said double stranded anchoring molecule or hairpin at a cleavage position upstream (5') of or adjacent to the complement of the nucleic acid tag sequence, thereby removing the complement of said tag sequence on said 3' strand.

8. A method according to claim 7 wherein cleavage is carried out by providing on said hairpin a recognition sequence for an endonuclease capable of cleaving the 3' strand of the anchoring molecule or hairpin at a cleavage site upstream of or adjacent to the complement of nucleic acid tag sequence to remove the complement of said tag sequence on said 3' strand and contacting with said endonuclease.

9. A method according to claim 7 or 8 wherein said cleavage step to generate the overhanging sequence is carried out prior to said sequencing and sequencing comprises adding one or more nucleotides simultaneously or sequentially to the 3' hydroxyl group generated by cleavage of the 3' strand and determining the identity of one or more of the added nucleotides.

10. A method according to claim 7 or claim 8 wherein sequencing of a portion of the target nucleic acid sequence is carried out prior to said cleavage step, said sequencing comprising adding one or more nucleotides simultaneously or sequentially to the 3' end of the anchor molecule or hairpin and determining the identity of one or more of the added nucleotides and sequencing of the nucleic acid sequence tag is carried out after said cleavage step said sequencing comprising adding one or more nucleotides simultaneously or sequentially to the 3' hydroxyl group generated by cleavage of the 3' strand and determining the identity of one or more of the added nucleotides.

11. A method according to claim 1 wherein said immobilised molecules are present on said array at a density of 10⁶-10⁹ immobilised molecules per cm2.

12. A method according to any one of the preceding claims wherein the nucleic acid sequence tag is from 1 to 10 nucleotides in length.

13. A method according to claim 12 wherein the nucleic acid sequence tag is 4, 5 or 6 nucleotides in length.

14. A method according to any of the preceding claims wherein said sequencing comprises at least one cycle of sequencing and is performed in the presence of a polymerase, said sequencing comprising addition of one or more nucleotides simultaneously or sequentially wherein each nucleotide comprises a characteristic label, and a blocking group that is capable of preventing uncontrolled polymerisation, wherein a cycle of sequencing comprises identifying any incorporated nucleotide incorporated by said polymerase and removing the blocking group and characteristic label from said nucleotide incorporated by said polymerase.

## Patentansprüche

1. Verfahren zum Sequenzieren und Unterscheiden zwischen Nukleinsäuresequenzen auf einem Einzelarray, wobei die Sequenzen von verschiedenen Quellen stammen, wobei das Verfahren die Schritte umfasst
a) Ligieren von Zielnukleinsäuresequenzen, die DNA-Polynukleotide aus einer ersten Quelle umfassen, an doppelsträngige Nukleinsäure-Ankermoleküle, wodurch markierte Zielnukleinsäuresequenzen erzeugt werden, worin die doppelsträngigen Nukleinsäure-Ankermoleküle eine Markierung der Nukleinsäuresequenz, die charakteristisch für die Quelle der Zielnukleinsäuresequenz ist und eine Funktionalität umfassen, die fähig ist, eine Immobilisierung an den Array zu bewirken, und worin das 3'-Ende jedes Strangs des DNA-Polynukleotids an das 5'-Ende eines Strangs des doppelsträngigen Ankermoleküls ligiert ist, dessen 5'-Ende nicht für die Verankerung verwendet wird;
b) Wiederholen von Schritt a) für Zielnukleinsäuresequenzen aus zweiten und nachfolgenden Quellen unter Verwendung von zweiten und nachfolgenden doppelsträngigen Nukleinsäure-Ankermolekülen, die charakteristische Markierungen der Nukleinsäuresequenz für jede der Quellen haben;
c) Immobilisieren von markierten Zielnukleinsäuresequenzen aus verschiedenen Quellen an den Array über die Funktionalität, die fähig ist, die Immobilisation zu bewirken, wodurch immobilisierte Moleküle erzeugt werden, wobei jedes immobilisierte Molekül eine Zielnukleinsäuresequenz und eine Markierung der Nukleinsäuresequenz umfasst, die charakteristisch für die Quelle der Zielnukleinsäuresequenz ist, worin die Nukleinsäuresequenzen auf dem Array mit einer Dichte angeordnet sind, so dass sie zur Einzelauflösung unter Verwendung von optischer Mikroskopie fähig sind; und
d) Sequenzieren der immobilisierten, einzeln auflösbaren Moleküle durch die Zugabe von Nukleotiden zu einer 3'-Hydroxylgruppe auf dem doppelsträngigen Anker, woraufhin das Sequenzieren eine Sequenz von jedem der Zielnukleinsäuremoleküle und die Markierungssequenz identifiziert, um die entsprechende Quelle des Zielnukleinsäuremoleküls zu identifizieren.

2. Verfahren nach Anspruch 1, worin das doppelsträngige Nukleinsäure-Ankermolekül ein Haarnadel-Oligonukleotid ist und das DNA-Polynukleotid an das 5'-Ende des Harrnadelnukleotids ligiert ist.

3. Verfahren nach Anspruch 1 oder Anspruch 2, worin das doppelsträngige Nukleinsäure-Ankermolekül oder die Haarnadel eine 5'-Überhangsequenz umfasst und die Markierung der Nukleinsäuresequenz auf der Überhangsequenz lokalisiert ist.

4. Verfahren nach Anspruch 2, worin das Haarnadel-Oligonukleotid eine einzelsträngige Nukleinsäuresequenz umfasst, die einen Bereich von interner Selbstkomplementarität enthält, wobei der Bereich fähig ist einen intramolekularen Duplex zu bilden.

5. Verfahren nach Anspruch 4, worin das Haarnadel-Oligonukleotid die Markierung der Nukleinsäuresequenz, die für die Quelle der Zielnukleinsäuresequenz charakteristisch ist, die direkt neben dem 5'-Ende der Haarnadel positioniert ist und das Komplement der Markierung der Nukleinsäuresequenz umfasst, das direkt neben dem 3'-Ende der Haarnadel positioniert ist.

6. Verfahren nach Anspruch 5, worin die Zielnukleinsäure an das Haarnadel-Oligonukleotid ligiert ist, indem jegliche Phosphatgruppen von den 5'- und 3'-Enden der Zielnukleinsäure entfernt werden, während eine einzelne Phosphatgruppe am 5'-Ende des Haarnadel-Oligonukleotids bereitgestellt wird und die Zielnukleinsäure und das Haarnadel-Oligonukleotid in Gegenwart eines Ligation-Reagenz inkubiert werden, wodurch ein 3'-Ende der Zielnukleinsäure an das 5'-Ende des Haarnadel-Oligonukleotids ligiert wird.

7. Verfahren nach einem der Ansprüche 3 bis 6, worin die Überhangsequenz in einem Spaltungsschritt erzeugt wird, der die Spaltung des 3'-Strangs des doppelsträngigen Ankermoleküls oder der Haarnadel an einer Spaltungsstelle umfasst, die vorgelagert (5') von oder neben dem Komplement der Nukleinsäuremarkierungssequenz ist, wodurch das Komplement der Markierungssequenz auf dem 3'-Strang entfernt wird.

8. Verfahren nach Anspruch 7, worin die Spaltung durchgeführt wird, indem auf der Haarnadel eine Erkennungssequenz für eine Endonuclease bereitgestellt wird, die fähig ist, den 3'-Strang des Ankermoleküls oder der Haarnadel an einer Spaltungsstelle, die vorgelagert von oder neben dem Komplement der Nukleinsäuremarkierungssequenz ist, zu spalten, um das Komplement der Markierungssequenz auf dem 3'-Strang zu entfernen und mit der Endonuclease in Kontakt gebracht wird.

9. Verfahren nach Anspruch 7 oder 8, worin der Spaltungsschritt, um die Überhangsequenz zu erzeugen, vor dem Sequenzieren durchgeführt wird und wobei das Sequenzieren die Zugabe eines oder mehrerer Nukleotids/Nukleotide gleichzeitig oder nacheinander zur 3'-Hydroxylgruppe, die durch die Spaltung des 3'-Strangs erzeugt wird, und die Bestimmung der Identität eines oder mehrerer der zugegebenen Nukleotide umfasst.

10. Verfahren nach Anspruch 7 oder Anspruch 8, worin das Sequenzieren eines Abschnitts der Zielnukleinsäuresequenz vor dem Spaltungsschritt durchgeführt wird, wobei das Sequenzieren die Zugabe eines oder mehrerer Nukleotids/Nukleotide gleichzeitig oder nacheinander an das 3'-Ende des Ankermoleküls oder der Haarnadel und die Bestimmung der Identität eines oder mehrerer der zugegebenen Nukleotide umfasst und wobei das Sequenzieren der Markierung der Nukleinsäuresequenz nach dem Spaltungsschritt durchgeführt wird, wobei das Sequenzieren die Zugabe eines oder mehrerer Nukleotids/Nukleotide gleichzeitig oder nacheinander zur 3'-Hydroxylgruppe, die durch die Spaltung des 3'-Strangs erzeugt wurde, und die Bestimmung der Identität eines oder mehrerer der zugegebenen Nukleotide umfasst.

11. Verfahren nach Anspruch 1, worin die immobilisierten Moleküle auf dem Array mit einer Dichte von 10⁶-10⁹ immobilisierte Moleküle pro cm2 vorhanden sind.

12. Verfahren nach einem der vorhergehenden Ansprüche, worin die Markierung der Nukleinsäuresequenz 1 bis 10 Nukleotide lang ist.

13. Verfahren nach Anspruch 12, worin die Markierung der Nukleinsäuresequenz 4, 5 oder 6 Nukleotide lang ist.

14. Verfahren nach einem der vorhergehenden Ansprüche, worin das Sequenzieren mindestens einen Zyklus Sequenzieren umfasst und in Gegenwart einer Polymerase durchgeführt wird, wobei das Sequenzieren die Zugabe eines oder mehrerer Nukleotids/Nukleotide gleichzeitig oder nacheinander umfasst, worin jedes Nukleotid eine charakteristische Markierung umfasst und eine Blockierungsgruppe, die fähig ist unkontrollierte Polymerisation zu verhindern, worin ein Zyklus Sequenzieren die Identifikation jedes eingebauten Nukleotids, das durch die Polymerase eingebaut wird, und das Entfernen der Blockierungsgruppe und der charakteristischen Markierung von dem Nukleotid, das durch die Polymerase eingebaut wird, umfasst.

## Revendications

1. Méthode pour séquencer et faire la différence entre des séquences d'acides nucléiques sur un réseau unique, séquences qui proviennent de sources différentes, méthode qui comprend les étapes
a) de ligation de séquences d'acides nucléiques cibles comprenant des polynucléotides d'ADN provenant d'une première source à des molécules d'ancrage d'acide nucléique bicaténaire en produisant ainsi des séquences d'acides nucléiques cibles marquées, lesdites molécules d'ancrage d'acide nucléique bicaténaire comprenant un marqueur de séquence d'acide nucléique caractéristique de la source de séquence d'acide nucléique cible et une fonctionnalité capable d'effectuer l'immobilisation audit réseau, et l'extrémité 3' de chaque brin dudit polynucléotide d'ADN étant réunie par ligation à l'extrémité 5' d'un brin de ladite molécule d'ancrage bicaténaire, extrémité 5' qui n'est pas utilisée pour l'ancrage ;
b) de répétition de l'étape a) pour des séquences d'acides nucléiques cibles provenant d'une seconde source et de sources suivantes en utilisant des secondes molécules d'ancrage d'acide nucléique bicaténaire et des molécules d'ancrage d'acide nucléique bicaténaire suivantes comprenant des marqueurs de séquence d'acide nucléique caractéristiques pour chacune desdites sources ;
c) d'immobilisation des séquences d'acides nucléiques cibles marquées provenant des différentes sources audit réseau par l'intermédiaire de la fonctionnalité capable d'effectuer l'immobilisation, en produisant ainsi des molécules immobilisées, chaque molécule immobilisée comprenant une séquence d'acide nucléique cible et un marqueur de séquence d'acide nucléique caractéristique de la source de séquence d'acide nucléique cible, lesdites séquences d'acides nucléiques sur ledit réseau étant disposées en une densité telle qu'elles soient aptes à la résolution individuelle en utilisant la microscopie optique ; et
d) de séquençage desdites molécules immobilisées aptes à la résolution individuelle par addition de nucléotides à un groupe 3'-hydroxyle sur l'ancre bicaténaire, ce qui fait que ledit séquençage identifie une séquence de chacune des molécules d'acides nucléiques cibles et la séquence du marqueur pour identifier la source correspondante de la molécule d'acide nucléique cible.

2. Méthode suivant la revendication 1, dans laquelle ladite molécule d'ancrage d'acide nucléique bicaténaire est un oligonucléotide en épingle à cheveux et ledit polynucléotide d'ADN est réuni par ligation à l'extrémité 5' dudit nucléotide en épingle à cheveux.

3. Méthode suivant la revendication 1 ou la revendication 2, dans laquelle ladite molécule d'ancrage bicaténaire ou ladite épingle à cheveux comprend une séquence en saillie 5' et le marqueur de séquence d'acide nucléique est situé sur ladite séquence en saillie.

4. Méthode suivant la revendication 2, dans laquelle ledit oligonucléotide en épingle à cheveux comprend une séquence d'acide nucléique monocaténaire qui contient une région d'autocomplémentarité interne, ladite région étant capable de former un duplex intramoléculaire.

5. Méthode suivant la revendication 4, dans laquelle ledit oligonucléotide en épingle à cheveux comprend ledit marqueur de séquence d'acide nucléique caractéristique de ladite source de séquence d'acide nucléique cible présent en position immédiatement adjacente à l'extrémité 5' de ladite épingle à cheveux et le complément dudit marqueur de séquence d'acide nucléique présent en position immédiatement adjacente à l'extrémité 3' de ladite épingle à cheveux.

6. Méthode suivant la revendication 5, dans laquelle ledit acide nucléique cible est réuni par ligation audit oligonucléotide en épingle à cheveux par élimination de n'importe quels groupes phosphates des extrémités 5' et 3' dudit acide nucléique cible tout en fournissant un groupe phosphate unique à l'extrémité 5' dudit oligonucléotide en épingle à cheveux, et en mettant en incubation ledit acide nucléique cible et ledit oligonucléotide en épingle à cheveux en présence d'un réactif de ligation, ce qui fait qu'une extrémité 3' de l'acide nucléique cible est réunie par ligation à l'extrémité 5' dudit oligonucléotide en épingle à cheveux.

7. Méthode suivant l'une quelconque des revendications 3 à 6, dans laquelle ladite séquence en saillie est engendrée dans une étape de clivage comprenant le clivage du brin 3' de ladite molécule d'ancrage bicaténaire ou de ladite épingle à cheveux à une position de clivage en amont (5') du, ou adjacente au, complément de la séquence de marqueur d'acide nucléique, en éliminant ainsi le complément de ladite séquence de marqueur sur ledit brin 3'.

8. Méthode suivant la revendication 7, dans laquelle le clivage est effectué en fournissant sur ladite épingle à cheveux une séquence de reconnaissance pour une endonucléase capable de cliver le brin 3' de la molécule d'ancrage ou de l'épingle à cheveux à un site de clivage en amont du, ou adjacent au, complément de la séquence de marqueur d'acide nucléique pour éliminer le complément de ladite séquence de marqueur sur ledit brin 3' et en mettant en contact avec ladite endonucléase.

9. Méthode suivant la revendication 7 ou 8, dans laquelle ladite étape de clivage pour engendrer la séquence en saillie est mise en oeuvre avant ledit séquençage et le séquençage comprend l'addition d'un ou plusieurs nucléotides simultanément ou séquentiellement au groupe 3'-hydroxyle engendré par clivage du brin 3' et la détermination de l'identité d'un ou plusieurs des nucléotides ajoutés.

10. Méthode suivant la revendication 7 ou la revendication 8, dans laquelle le séquençage d'une portion de la séquence d'acide nucléique cible est effectué avant ladite étape de clivage, ledit séquençage comprenant l'addition d'un ou plusieurs nucléotides simultanément ou séquentiellement à l'extrémité 3' de la molécule d'ancrage ou de l'épingle à cheveux et la détermination de l'identité d'un ou plusieurs des nucléotides ajoutés et le séquençage du marqueur de séquence d'acide nucléique est effectué après ladite étape de clivage, ledit séquençage comprenant l'addition d'un ou plusieurs nucléotides simultanément ou séquentiellement au groupe 3'-hydroxyle engendré par clivage du brin 3' et la détermination de l'identité d'un ou plusieurs des nucléotides ajoutés.

11. Méthode suivant la revendication 1, dans laquelle lesdites molécules immobilisées sont présentes sur ledit réseau en une densité de 10⁶ à 10⁹ molécules immobilisées par cm²_{.}

12. Méthode suivant l'une quelconque des revendications précédentes, dans laquelle le marqueur de séquence d'acide nucléique a une longueur de 1 à 10 nucléotides.

13. Méthode suivant la revendication 12, dans laquelle le marqueur de séquence d'acide nucléique a une longueur de 4, 5 ou 6 nucléotides.

14. Méthode suivant l'une quelconque des revendications précédentes, dans laquelle ledit séquençage comprend au moins un cycle de séquençage et est effectué en présence d'une polymérase, ledit séquençage comprenant l'addition d'un ou plusieurs nucléotides simultanément ou séquentiellement, chaque nucléotide comprenant un marqueur caractéristique, et d'un groupe de blocage qui est capable d'empêcher une polymérisation incontrôlée, dans laquelle un cycle de séquençage comprend l'identification de n'importe quel nucléotide incorporé, qui a été incorporé par ladite polymérase, et l'élimination du groupe de blocage et du marqueur caractéristique dudit nucléotide incorporé par ladite polymérase.
